# FASCICULE DE BREVET EUROPEEN

(11) **EP 0 625 345 B1**
(45) Date de publication et mention de la délivrance du brevet: **02.06.1999**
(21) Numéro de dépôt: 94400950.5
(22) Date de dépôt: 03.05.1994
(51) Int. Cl.: A61F 13/15

(54) **Serviette périodique formant enveloppe de protection avant utilisation**
Eine Monatsbinde, die vor der Anwendung zu einem Umschlag gefaltet ist
Sanitary towel forming a protection enveloppe before the use

(30) Priorité: 14.05.1993 FR 9305830
(43) Date de publication de la demande: 23.11.1994
(73) Titulaire: FORT JAMES FRANCE, 68320 Kunheim (FR)
(72) Inventeur: Lefebvre du Grosriez, Carol, F-78600 Maisons Lafitte (FR)
(74) Mandataire: David, Daniel

(56) Documents cités:
- EP-A- 0 357 000
- WO-A-89/02728
- US-A- 3 973 567
- US-A- 4 608 047

## Description

L'invention concerne un article d'hygiène féminine et plus précisément une serviette périodique formant enveloppe de protection avant utilisation et éventuellement conditionnement ou sachet jetable après utilisation.

Les serviettes périodiques actuellement sur le marché sont fournies généralement dans un emballage ou conditionnement individuel sous forme de sachet séparé dans lequel est introduite la serviette périodique avant, et après utilisation pour être jetée.

Or, le sachet individuel formant emballage nécessite la fabrication d'un élément supplémentaire, ce qui augmente considérablement les coûts de production.

Un tel produit comprend plusieurs éléments à manipuler au cours de son utilisation.

En effet, pour un article comprenant par exemple un sachet individuel ou "pochette service" et une serviette périodique munie de volets latéraux, il est nécessaire pour utiliser la serviette périodique de la sortir de son sachet individuel, de retirer successivement la bande disposée sous la partie centrale absorbante de la serviette et les autres petites bandes siliconées disposées sous chacun des volets latéraux alors que l'utilisatrice ne dispose que de deux mains. De même, lors du changement de protection, la serviette usagée est un élément supplémentaire à prendre en compte dans la manipulation.

Des serviettes formant enveloppe ou sachet jetable ont déjà été proposées et décrites dans différents brevets.

Le brevet américain n° 4 608 047 révèle une serviette périodique comprenant des volets latéraux destinés à être rabattus sous un sous-vêtement pour maintenir la serviette en place. De manière avantageuse, un dépôt d'adhésif est effectué sur un des volets pour fixer ce volet à l'autre volet lorsqu'ils sont rabattus sous le sous-vêtement.

Afin de former une enveloppe ou sachet jetable, la serviette, plus précisément l'élément central absorbant de la serviette est plié en trois, puis les volets sont rabattus l'un après l'autre sur la serviette pliée et recouvrent totalement cette dernière. Les volets une fois pliés se fixent l'un à l'autre par le dépôt d'adhésif prévu sur l'un d'entre eux. La serviette est ainsi totalement refermée. Mais l'élément central qui vient se poser sur le sous-vêtement comprend également sur sa face imperméable au contact du sous-vêtement, des bandes d'adhésifs, si bien qu'il est nécessaire d'appliquer un élément de protection du type bande siliconée sur ces bandes d'adhésifs, avant de replier l'élément central si la serviette est pliée pour former son propre conditionnement avant utilisation.

Dans ce mode de réalisation, l'élément de protection disposé sous l'élément central est de dimension sensiblement égale à celle de cet élément central et doit être appliqué au cours de la fabrication de la serviette, avant pliage de l'élément central. De ce fait, pour ouvrir la serviette, il est nécessaire dans un premier temps de séparer les volets l'un de l'autre, et dans un second temps, de dégager le moyen de protection situé sous l'élément central.

La demande de brevet français n° 2 620 936 a pour objet une serviette périodique comprenant des volets latéraux conçus de façon à former, après emploi de la serviette périodique, par repliement autour de la serviette périodique elle-même repliée, un conditionnement ou sachet jetable. Les volets latéraux sont également prévus pour former une partie intégrante d'une pochette de protection dans laquelle est conditionnée la serviette avant emploi.

Or, dans un tel mode de réalisation, lorsque la serviette est pliée pour former un emballage avant utilisation, des protections telles que des petites bandes siliconées sont nécessaires pour protéger les moyens de fixation tels que des pavés d'adhésifs disposés en croix sur la face inférieure des volets et de la partie centrale de la serviette destinée à être posée sur un sousvêtement.

Quant au brevet européen n° 0 299 532, il décrit une serviette périodique pouvant être pliée ou roulée et auto-collée afin d'être jetée, comprenant deux rabats s'étendant transversalement au-delà du bord longitudinal de la couche de recouvrement, elle-même située sous le matelas absorbant et face au sous-vêtement. Les rabats sont situés à une extrémité de la couche de recouvrement et comportent des moyens adhésifs. Afin d'être jetée, la serviette est roulée ou pliée en deux et les rabats sont pliés sur la couche de recouvrement de la serviette pliée, pour envelopper cette dernière. Ce mode de réalisation n'est pas prévu pour un emballage de la serviette sous forme d'enveloppe, avant utilisation.

L'invention a pour but de fournir une serviette périodique qui forme une enveloppe de protection avant emploi et qui peut former un conditionnement jetable après usage de la serviette.

L'invention a aussi pour but de fournir une serviette périodique qui permet l'utilisation d'un moyen unique de protection des moyens de fixation tels que des dépôts d'adhésifs, ce moyen de protection étant de plus de dimension réduite.

L'invention a encore pour but de fournir une serviette périodique qui, pliée et munie de son moyen de protection, forme une enveloppe protégeant la surface de la serviette qui sera au contact du corps.

L'invention a également pour but de réduire le nombre d'éléments qui s'individualisent et sont à manipuler au cours de l'utilisation de cette serviette.

L'invention résout les problèmes des serviettes formant enveloppe ou sachet jetable de l'art antérieur, en ayant pour objet une serviette périodique formant enveloppe de protection avant utilisation et éventuellement conditionnement jetable après usage, du type comprenant un élément central absorbant les fluides, disposé dans une enveloppe comportant sur sa face au contact du sous-vêtement des moyens de fixation audit sous-vêtement et au moins un volet latéral disposé le long dudit élément central, pouvant être rabattu sous le sous-vêtement et muni d'au moins un moyen de fixation audit sous-vêtement, caractérisée en ce que ladite enveloppe de protection ou conditionnement formé par la serviette avant et après usage de celle-ci, est constitué par le repli dudit volet sur l'élément central lui-même plié en trois ou en C, l'élément central replié ne chevauchant pas ses propres moyens de fixation et ledit volet une fois plié, ne chevauchant pas les moyens de fixation dudit élément central plié et le cas échéant, d'un autre volet.

Selon une caractéristique avantageuse de l'invention, la serviette périodique comprend deux volets latéraux.

Selon une autre caractéristique avantageuse de l'invention, la serviette en position repliée comprend un moyen de protection disposé sur les moyens de fixation à la fois de l'élément central et du ou des volets.

Selon une caractéristique préférentielle de l'invention, les moyens de fixation précités sont des pavés d'adhésifs et le moyen de protection précité est une bande siliconée.

La serviette selon l'invention est d'une utilisation très simple, car présentée sous forme pliée avant utilisation, il suffit pour l'ouvrir de retirer le moyen de protection, tous les moyens de fixation étant simultanément dégagés. Ceci n'était pas possible avec les serviettes de l'art antérieur, décrites précédemment.

D'autres caractéristiques et avantages de l'invention apparaîtront plus clairement à la lecture de la description qui suit en référence aux dessins annexés dans lesquels :
- la figure 1 représente une vue de dessus de la serviette périodique selon l'invention, c'est-à-dire de la face qui sera au contact du corps,
- la figure 2 représente une vue de dessous de la serviette périodique représentée à la figure 1, c'est-à-dire de la face qui sera au contact du sous-vêtement,
- la figure 3 est une vue en perspective de la serviette périodique selon l'invention montrant la manière dont elle est pliée,
- la figure 4 est une vue en perspective d'une serviette selon l'invention, totalement repliée formant enveloppe de protection sans moyen de protection disposé sur les moyens de fixation,
- la figure 5 est une vue similaire à celle de la figure 4 où la serviette pliée comprend de plus le moyen de protection refermant l'enveloppe de protection,
- la figure 6 est une vue en perspective d'une serviette repliée sans moyen de protection suivant un autre mode de réalisation de l'invention,
- la figure 7 représente une vue en perspective d'une serviette à l'état dépliée selon encore un autre mode de réalisation de l'invention, et
- la figure 8 est une vue en perspective de la serviette représentée en figure 7, à l'état replié.

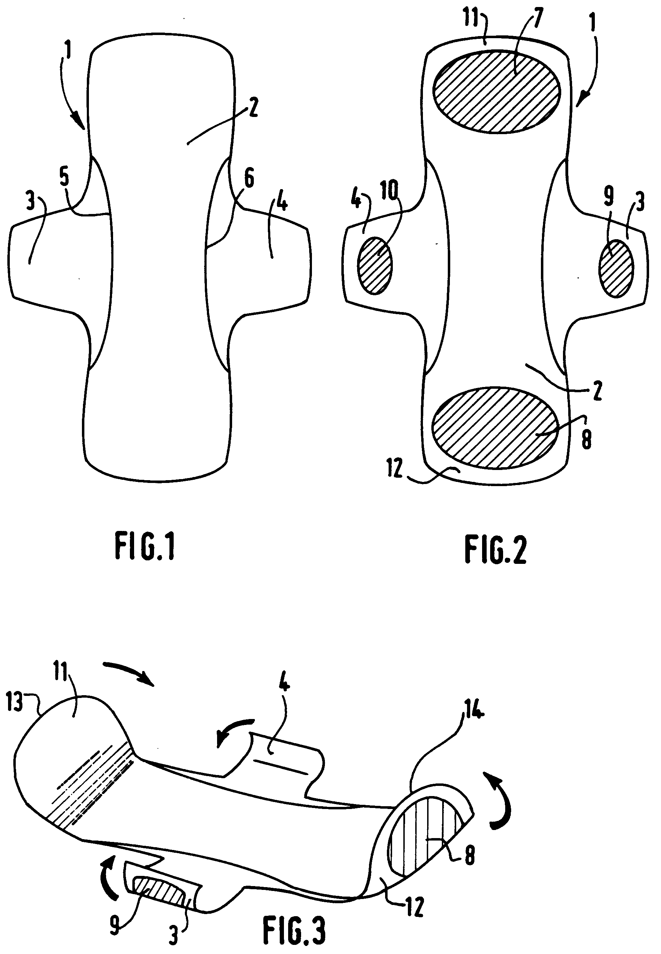

En référence aux figures 1 et 2, la serviette périodique 1 représentée à plat, comprend un élément central absorbant 2, tel qu'un matelas de fibres cellulosiques ou une enveloppe contenant des superabsorbants, et deux volets latéraux 3 et 4 disposés de part et d'autre des bords longitudinaux 5 et 6 de l'élément central 2.

Comme toute serviette périodique classique, l'élément central absorbant est disposé dans une enveloppe. Celle-ci peut être composée par exemple d'une face ou couche supérieure perméable aux fluides sous la forme d'un voile nontissé ou d'un voile plastique perforé, et d'une face ou couche inférieure imperméable aux fluides.

Les volets peuvent être une extension latérale de la couche inférieure imperméable située sous l'élément central absorbant ou encore une extension latérale de l'enveloppe de l'élément central mais sans matelas absorbant. Dans encore un autre mode de réalisation, les volets peuvent comporter, le cas échéant, une matière absorbante pour éviter toute fuite occasionnelle sur le sous-vêtement.

Les volets peuvent donc faire partie intégrante de la serviette avec l'élément central. Ils peuvent aussi être fabriqués à part et être rapportés ensuite sur l'élément central par tout mode de fixation approprié tel que le thermosoudage.

Des moyens de fixation ou d'attache sont appliqués en croix au dos de la serviette périodique dépliée ou à plat, sur la face de l'enveloppe au contact du sous-vêtement.

Les moyens de fixation 7 et 8 sont disposés sous l'élément central absorbant 2 de la serviette et les moyens de fixation 9 et 10 sont disposés sous les volets latéraux 3 et 4.

Les moyens de fixation sont de préférence des dépôts d'adhésifs sous forme de pavés. Ils peuvent également être une partie d'attache du type de celle connue sous la marque Velcro, l'autre partie correspondant au moyen de protection.

Comme l'illustre la figure 2, les moyens de fixation 7 et 8 sous forme de pavés d'adhésifs sont appliqués sur des surfaces situées aux extrémités 11 et 12 de l'élément central 2.

La figure 3 illustre la manière dont est pliée la serviette périodique pour former une enveloppe de protection ou conditionnement. Les deux extrémités 11 et 12 sont repliées en trois, de préférence en C Sur l'élément central 2. Les bords 13 et 14 des extrémités respectivement 11 et 12 sont placés de préférence l'un contre l'autre lorsque ces mêmes extrémités 11 et 12 sont complètement rabattues. Puis, les volets 3 et 4 sont repliés sur l'élément central 2 déjà replié. La couche supérieure perméable aux fluides est alors située à l'intérieur de la serviette pliée.

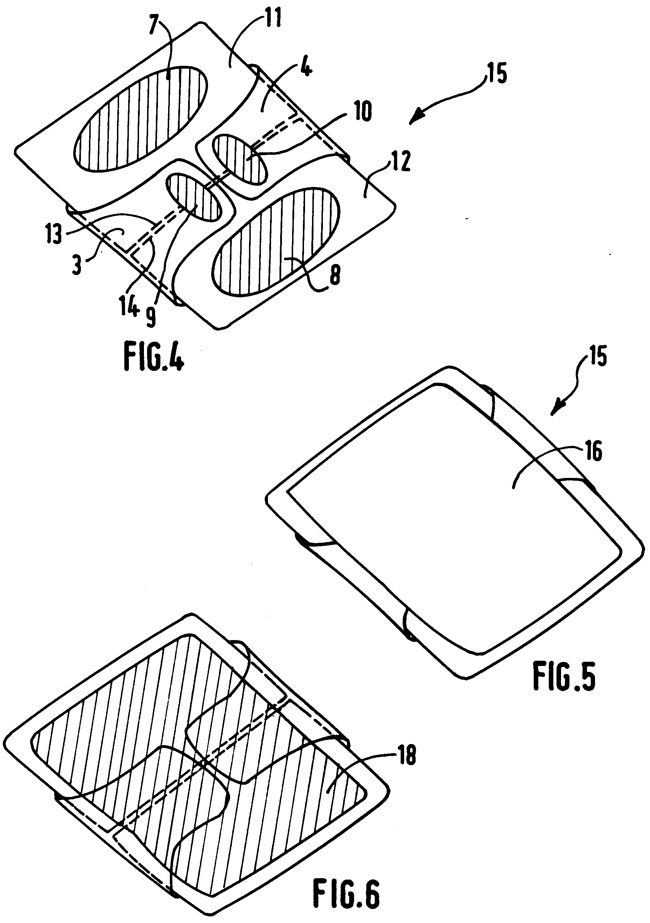

La figure 4 montre une serviette complètement repliée où les volets 3 et 4 sont repliés bord à bord sur l'élément central replié en C.

Le mode de pliage et la taille des éléments pliés sont prévus de telle manière que les extrémités 11 et 12 repliées de l'élément central 2 ne chevauchent pas ses propres pavés d'adhésifs, respectivement 8 et 7 et que les volets 3 et 4 repliés pour former une enveloppe de protection 15, ne chevauchent pas d'une part les pavés d'adhésifs 7 et 8 des extrémités 11 et 12 de l'élément central 2, de la serviette et d'autre part, ses propres pavés d'adhésifs respectivement 10 et 9.

De par ce mode de pliage et la taille des éléments repliés, chacun des moyens de fixation, ici des pavés d'adhésifs, fait partie d'un même plan correspondant à une face de la serviette 1 pliée ou de l'enveloppe de protection ou conditionnement 15.

Comme l'illustre la figure 5, un moyen de protection 16 pour l'ensemble des moyens adhésifs 7, 8, 9 et 10 est appliqué sur la serviette pliée qui est représentée en figure 4.

Ce moyen de protection 16 est ici une bande siliconée de dimension suffisante pour recouvrir tous les pavés d'adhésifs, et de préférence de dimension sensiblement égale à celle de la face de la serviette complètement repliée présentant à sa surface les pavés d'adhésifs.

Ainsi, il suffit de détacher la bande siliconée 16 pour ouvrir l'enveloppe 15 et libérer les moyens de fixation 7 8, 9 et 10, puis de déplier les volets 3 et 4 et l'élément central 2 pour que la serviette 1 soit prête à l'emploi.

De même, après usage, la serviette est repliée en suivant les mêmes opérations que celles décrites précédemment et l'on applique une bande siliconée pour refermer la serviette sous forme d'enveloppe ou conditionnement et la jeter de manière hygiénique. La serviette selon l'invention supprime tout emballage supplémentaire individuel.

Avant utilisation, la couche perméable située à l'intérieur de l'enveloppe de protection repliée et qui sera au contact du corps, est protégée de l'extérieur. En d'autres termes, la surface perméable de la serviette, lorsque celle-ci est en position repliée, n'est en contact avec aucun corps étranger ou extérieur à la serviette.

Suivant une variante de réalisation représentée à la figure 6, quasiment toute la face de la serviette pliée est encollée sous la forme d'un grand rectangle de colle 18.

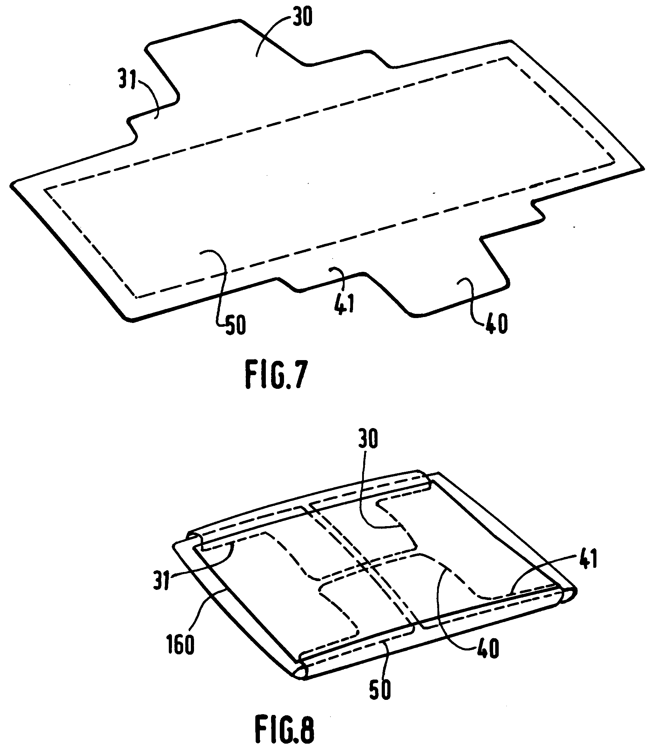

Suivant encore un autre mode de réalisation représenté aux figures 7 et 8, la découpe des volets 30 et 40 est différente du mode de réalisation décrit et représenté précédemment. En effet, les volets 30 et 40 présentent respectivement une extension latérale 31 et 41 le long du bord de l'élément central 50, si bien que lorsque les volets sont repliés sur l'élément central 50 déjà replié, ils enveloppent et recouvrent mieux cet élément central 50 plié. De ce fait, la surface de l'élément central est avantageusement protégée. Une fois le moyen de protection 160 disposé sur les volets 30 et 40 et l'élément central 50 repliés, l'introduction de petits objets ou particules à l'intérieur de la serviette pliée est complètement évitée.

Le procédé de fabrication du produit selon l'invention est très simplifié comparativement à ceux des serviettes formant sachet jetable de l'art antérieur.

Il consiste à assembler les différents éléments de la serviette périodique ce qui est bien connu de l'état de la technique, à plier en trois ou en C l'élément central puis à replier les volets latéraux autour de l'élément central plié, à appliquer les différents pavés d'adhésifs ou le rectangle d'adhésif sur la face de l'enveloppe de protection comportant les extrémités de l'élément central repliées et les volets repliés, et à déposer la bande siliconée. Les serviettes pliées sous forme d'enveloppe sont ensuite regroupées pour être conditionnées en paquets.

L'invention comprend tous les moyens équivalents et supplémentaires à la portée de l'homme du métier.

## Revendications

1. Serviette périodique (1) formant enveloppe de protection (15) avant utilisation et éventuellement conditionnement jetable après usage, du type comprenant un élément central absorbant les fluides (2) disposé dans une enveloppe comportant sur sa face au contact du sous-vêtement des moyens de fixation (7,8) audit sous-vêtement, et au moins un volet latéral (3) disposé le long dudit élément central, pouvant être rabattu sous le sous-vêtement et muni d'au moins un moyen de fixation (9) audit sous-vêtement, caractérisée en ce que ladite enveloppe de protection (15) ou conditionnement formé par la serviette (1) avant et après usage de celle-ci, est constitué par le repli dudit volet (3) sur ledit élément central (2) lui-même plié en trois ou en C, l'élément central (2) replié ne chevauchant pas ses propres moyens de fixation (7, 8) et ledit volet (3) une fois plié, ne chevauchant pas les moyens de fixation précités (7, 8) dudit élément central et le cas échéant, d'un autre volet.

2. Serviette périodique selon la revendication 1, caractérisée en ce qu'elle comprend deux volets latéraux (3 et 4) munis respectivement des moyens de fixation (9 et 10).

3. Serviette périodique selon la revendication 1 ou 2, caractérisée en ce qu'elle comprend en position repliée, un moyen de protection (16) disposé sur les moyens de fixation précités (7, 8, 9 et 10) à la fois de l'élément central et du ou des volets.

4. Serviette périodique selon l'une des revendications précédentes, caractérisée en ce que les moyens de fixation précités sont des pavés d'adhésifs et en ce que le moyen de protection précité est une bande siliconée.

5. Serviette périodique selon l'une des revendications 2 à 4, caractérisée en ce que les deux extrémités (13, 14) de l'élément central (2) et les deux volets latéraux (3, 4) sont repliés bord à bord.

## Claims

1. Sanitary towel (1) forming a protective envelope (15) before use and if needs be disposable packaging after use, of the type comprising a central element absorbing fluids (2) disposed in an envelope having on its face in contact with the undergarment means of attachment (7,8) to the said undergarment, and at least one side flap (3) disposed along the said central element, which can be turned back under the undergarment and provided with at least one means of attachment (9) to the said undergarment, characterized in that the said protective or packaging element (15) formed by the towel (1) before and after use thereof, is constituted by folding the said flap (3) onto the said central element (2), which is itself folded in three or into a C, the folded central element (2) not overlapping its own means of attachment (7,8) and the said flap (3), once folded, not overlapping the aforementioned means of attachment (7,8) of the said central element and, as the case may be, of another flap.

2. Sanitary towel according to claim 1, characterized in that it includes two side flaps (3 and 4) provided respectively with means of attachment (9 and 10).

3. Sanitary towel according to claim 1 or 2, characterized in that in the folded position it includes means of protection (16) disposed on the aforementioned means of attachment (7, 8, 9 and 10) both of the central element and of the flap or flaps.

4. Sanitary towel according to one of the preceding claims, characterized in that the aforementioned means of attachment are adhesive patches and in that the aforementioned means of protection is a siliconized strip.

5. Sanitary towel according to one of claims 2 to 4, characterized in that the two ends (13, 14) of the central element (2) and the two side flaps (3, 4) are folded edge-to-edge.

## Patentansprüche

1. Monatsbinde (1), die eine Schutzhülle (15) vor Gebrauch und gegebenenfalls eine nach Gebrauch wegwerfbare Verpackung bildet, mit einem flüssigkeitsabsorbierenden zentralen Element (2), das in einer Hülle angeordnet ist, die auf ihrer mit der Unterwäsche in Berührung stehenden Seite Befestigungsmittel (7, 8) zur Befestigung an der Unterwäsche aufweist, und mindestens einer seitlichen Lasche (3), die längs des zentralen Elementes angeordnet, unter die Unterwäsche zurückschlagbar und mit mindestens einem Befestigungsmittel (9) zur Befestigung an der Unterwäsche versehen ist, dadurch gekennzeichnet, daß die von der Binde (1) vor und nach Gebrauch gebildete Schutzhülle (15) oder Verpackung daraus besteht, daß die Lasche (3) auf das zentrale Element (2) gefaltet ist, das seinerseits drei- oder C-förmig gefaltet ist, wobei das gefaltete zentrale Element (2) seine eigenen Befestigungsmittel (7, 8) nicht überdeckt und die einmal gefaltete Lasche (3) die Befestigungsmittel (7, 8) des zentralen Elementes und gegebenenfalls einer weiteren Lasche nicht überdeckt.

2. Monatsbinde nach Anspruch 1, dadurch gekennzeichnet, daß sie zwei seitliche Laschen (3 und 4) aufweist, die mit entsprechenden Befestigungsmitteln (9 und 10) versehen sind.

3. Monatsbinde nach Anspruch 1 oder 2, dadurch gekennzeichnet, daß sie im gefalteten Zustand ein Schutzmittel (16) aufweist, das über den besagten Befestigungsmitteln (7, 8, 9 und 10) sowohl des zentralen Elementes wie auch der Lasche bzw. der Laschen angeordnet ist.

4. Monatsbinde nach einem der vorhergehenden Ansprüche, dadurch gekennzeichnet, daß die besagten Befestigungsmittel Klebestreifen sind und daß das besagte Schutzmittel ein Siliconband ist.

5. Monatsbinde nach einem der Ansprüche 2 bis 4, dadurch gekennzeichnet, daß die beiden Enden (13, 14) des zentralen Elementes (2) und die beiden seitlichen Laschen (3, 4) so gefaltet sind, daß ihre Ränder aneinander stoßen.
